# FASCICULE DE BREVET EUROPEEN

(11) **EP 2 059 583 B1**
(45) Date de publication et mention de la délivrance du brevet: **19.07.2017**
(21) Numéro de dépôt: 07823459.8
(22) Date de dépôt: 30.08.2007
(51) Int. Cl.: G01N 1/22, C12M 1/26

(54) **DISPOSITIF DE COLLECTE ET DE SEPARATION DE PARTICULES ET DE MICROORGANISMES PRESENTS DANS L'AIR AMBIANT**
VORRICHTUNG ZUR SAMMLUNG UND TRENNUNG VON PARTIKELN UND MIKROORGANISMEN IN DER UMGEBUNGSLUFT
DEVICE FOR COLLECTING AND SEPARATING PARTICLES AND MICROORGANISMS PRESENT IN THE AMBIENT AIR

(30) Priorité: 04.09.2006 FR 0607729
(43) Date de publication de la demande: 20.05.2009
(73) Titulaire: Bertin Technologies S.A., 78180 Montigny-le-Bretonneux (FR)
(72) Inventeur: VERDIER, Amandine, 92240 Malakoff (FR); SOREL, Emmanuelle, 28260 Rouvres (FR); VALLAYER, Bruno, 33370 Yvrac (FR); REBUFFAT, Denis, 64600 Anglet (FR)
(74) Mandataire: Ramey, Daniel
(86) Numéro de dépôt international: PCT/FR2007/001414
(87) Numéro de publication internationale: WO 2008/029014

(56) Documents cités:
- FR-A1- 2 855 831
- US-A- 5 412 975
- US-A- 5 902 385
- US-A1- 2004 173 034

## Description

L'invention concerne un dispositif de collecte et de séparation de particules et de microorganismes présents dans l'air ambiant, à des fins d'identification et de dénombrement de ces particules et microorganismes.

Cette identification et ce dénombrement sont importants dans de nombreux domaines tels que l'industrie pharmaceutique, l'industrie agroalimentaire, le milieu médical, les services d'hygiène, les services vétérinaires, la surveillance de sites, etc, les dimensions des particules et microorganismes à collecter pouvant varier de 0,5µm à plusieurs dizaines de microns.

On connaît, par le document FR-A-2 855 831, un dispositif de ce type qui comprend une enceinte amovible de centrifugation associée à des moyens d'aspiration d'air, l'enceinte comprenant une entrée et une sortie d'air et formant un récipient de transport d'un échantillon liquide contenant les particules et microorganismes collectés.

Il est souhaitable de faire varier les caractéristiques de prélèvement des particules et microorganismes en fonction des conditions dans lesquelles ces prélèvements sont réalisés et par exemple :
- à l'intérieur ou à l'extérieur de locaux,
- à des fins de surveillance continue ou sur une longue période ou en cas d'alerte,
- en fonction des caractéristiques des microorganismes collectés.

Il est notamment souhaitable, en cas d'alerte, de réaliser le prélèvement avec un débit élevé d'aspiration, pour avoir un résultat aussi rapidement que possible. A l'inverse, un prélèvement à des fins de surveillance peut être réalisé avec un débit plus faible et sur une plus longue période.

De même, des débits d'aspiration relativement faibles seront mieux appropriés à la préservation de certains microorganismes.

Il n'existe pas actuellement d'appareils de collecte et de séparation de particules et de microorganismes présents dans l'air ambiant, dont on puisse faire varier les débits d'aspiration dans une large mesure et dans des conditions préservant la qualité et la fiabilité des prélèvements réalisés.

L'invention a notamment pour but de répondre à ce besoin.

Elle propose à cet effet un dispositif de collecte et de séparation de particules et de microorganismes présents dans l'air ambiant, comprenant un ensemble d'enceintes amovibles de centrifugation comportant chacune une entrée et une sortie d'air et formant un récipient de transport d'un échantillon liquide contenant les particules et microorganismes collectés, le dispositif comportant en outre des moyens d'aspiration d'air dans l'une desdites enceintes, caractérisé en ce que ladite enceinte est sélectionnée dans l'ensemble d'enceintes du dispositif ayant des diamètres différents et permettant de réaliser, avec les mêmes moyens d'aspiration, des prélèvements avec des débits d'aspiration compris entre 100 et 2000 litres par minute, environ.

C'est en effet en faisant varier le diamètre des enceintes de centrifugation que l'on peut faire varier les débits d'aspiration dans une large mesure tout en assurant la qualité et la fiabilité des prélèvements.

Dans un mode de réalisation de l'invention, les diamètres des enceintes sont compris entre 20 et 100 mm environ, pour des débits d'aspiration compris entre 100 et 2000 litres par minute environ.

Chaque enceinte de centrifugation comprend une partie supérieure cylindrique reliée à une entrée d'air et une partie inférieure conique dans laquelle s'accumule le liquide de prélèvement. Pour conserver une quantité de liquide de prélèvement à peu près identique dans chaque enceinte et une même efficacité de séparation quelle que soit la dimension de l'enceinte, il est avantageux que l'extrémité inférieure de la partie conique d'au moins la plus grande enceinte soit tronquée.

Selon d'autres caractéristiques de l'invention:
- l'enceinte est raccordée par une canne de prélèvement à un boîtier comprenant les moyens d'aspiration d'air, ce boîtier pouvant être disposé horizontalement ou verticalement en position de service,
- le dispositif comprend des moyens de détermination et/ou de contrôle de la quantité de liquide dans l'enceinte et des moyens d'injection de liquide dans l'enceinte,
- le dispositif comprend des moyens de mesure du débit d'air aspiré dans l'enceinte, ces moyens comprenant un capteur de pression monté sur la canne de prélèvement reliant l'enceinte aux moyens d'aspiration d'air,
- le point de montage du capteur de pression sur la canne de prélèvement est éloigné du coude de la canne du côté relié à l'enceinte et de l'entrée des moyens d'aspiration,
- le dispositif comprend au moins un flacon de recueil de prélèvement, placé à l'intérieur d'un compartiment refroidi, par exemple équipé d'un élément thermoélectrique à effet Peltier,
- l'enceinte comprend à son extrémité inférieure des moyens de connexion au flacon de recueil,
- les enceintes sont équipées d'entrées d'air amovibles, qui peuvent être du type unidirectionnel ou omnidirectionnel.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- les figures 1 a et 1 b, 2a et 2b et 3a, 3b sont des vues schématiques en élévation et en coupe axiale respectivement d'enceintes de prélèvement de dimensions différentes ;
- la figure 4 est un graphe représentant les débits d'aspiration que l'on peut atteindre, avec un même moteur, pour des enceintes de dimensions différentes ;
- la figure 5 représente schématiquement la forme générale d'une enceinte dont l'extrémité inférieure a été tronquée ;
- la figure 6 est une vue schématique d'un dispositif selon l'invention équipé d'un capteur de pression pour la mesure du débit d'aspiration.

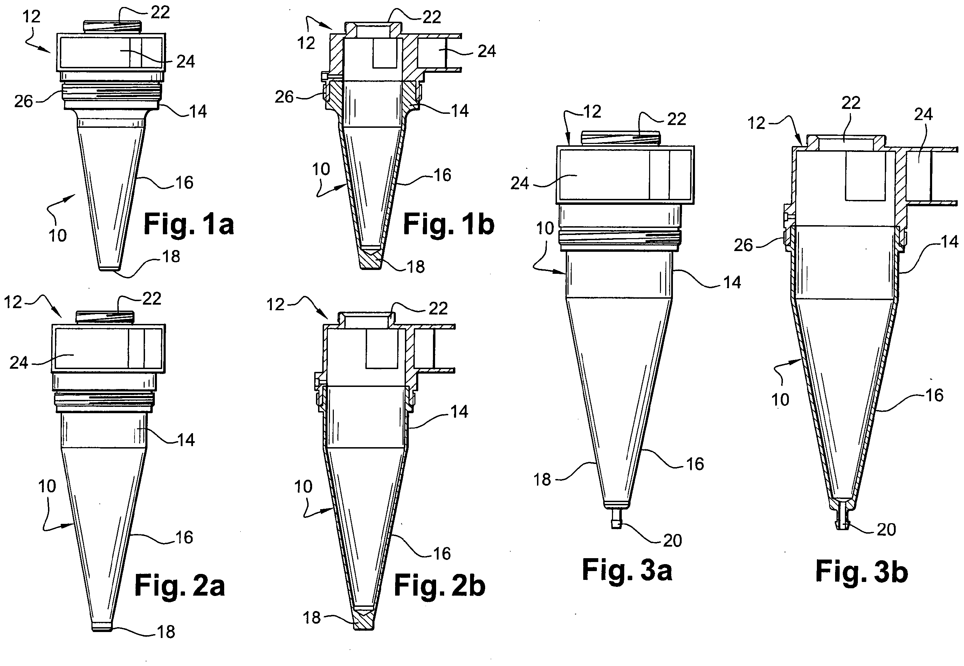

Dans les figures 1a à 3b, les enceintes de centrifugation 10 sont représentées associées à des éléments 12 d'entrée et de sortie d'air et ont des diamètres différents, l'enceinte 10 des figures 1a et 1b ayant un diamètre de 30mm, celles des figures 2a et 2b un diamètre de 40mm et celles des figures 3a et 3b un diamètre de 50mm.

L'élément 12 d'entrée et de sortie d'air vient coiffer une partie cylindrique supérieure 14 de l'enceinte, raccordée à une partie inférieure conique 16 dont la section diminue jusqu'à son extrémité inférieure 18 qui peut être fermée comme représenté pour les deux plus petites enceintes des figures 1a, 1b, 2a et 2b ou qui peut être munie d'un tube ou d'un clapet 20 de sortie de liquide comme représenté pour l'enceinte des figures 3a et 3b, pour la vidange de l'enceinte dans un flacon de recueil, le tube de sortie 20 comportant des moyens d'ouverture et de fermeture tels qu'une pince, une électrovanne, etc.

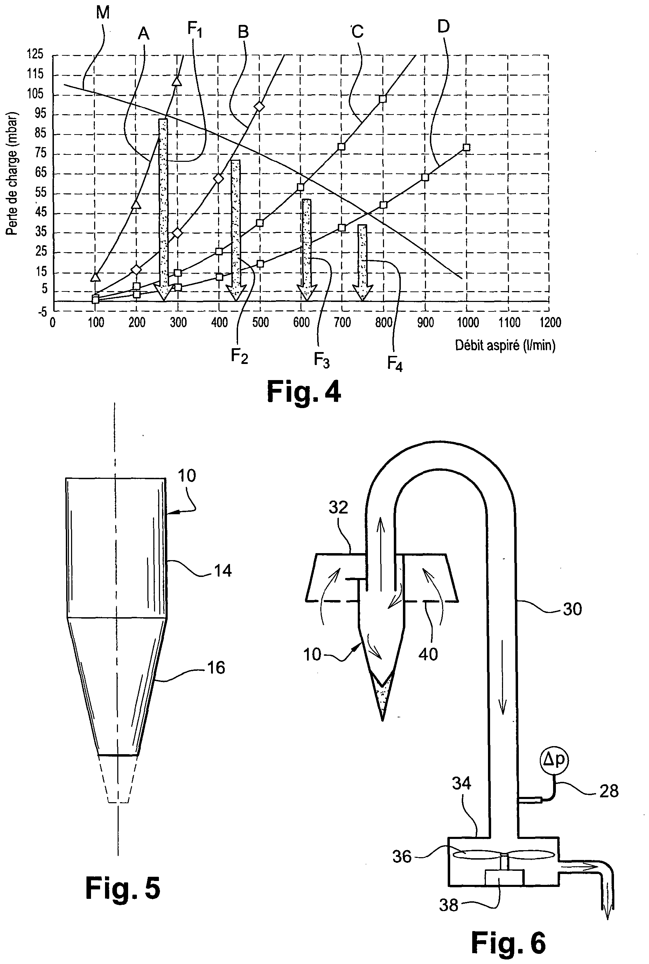

L'élément 12 d'entrée et de sortie d'air qui équipe chaque enceinte est ici à entrée unidirectionnelle et comporte une paroi supérieure transversale formée avec un embout axial 22 de raccordement à une canne de prélèvement en forme de J (représentée schématiquement en figure 6), une paroi cylindrique formée avec une entrée d'air 24 tangentielle et une extrémité inférieure ouverte, venant coiffer la partie cylindrique supérieure 14 de l'enceinte 10 et comportant une jupe cylindrique 26 de fixation amovible sur la partie supérieure 14 de l'enceinte 10. La fixation de l'élément 12 d'entrée et de sortie d'air sur l'enceinte 10 peut se faire de toute façon appropriée, par exemple par vissage rapide, par un montage à baïonnette, par encliquetage élastique, etc.

Les débits d'aspiration que l'on peut atteindre avec ces enceintes sont représentés en figure 4, dans laquelle les débits d'air aspirés sont portés en abscisse et la perte de charge en ordonnée, celle-ci se produisant essentiellement dans l'entrée d'air 24 de l'enceinte.

Les débits d'aspiration atteignables sont fonction du moyen d'aspiration utilisé et peuvent être déterminés à partir de la courbe caractéristique M du moteur d'aspiration et des caractéristiques aérauliques des enceintes. En figure 4, la courbe A représente la perte de charge en fonction du débit aspiré pour l'enceinte 10 des figures 1a et 1b, d'un diamètre de 30mm et la flèche F1 indique le débit atteignable pour cette enceinte, soit environ 280 litres par minute.

De même, la courbe B représente la variation de la perte de charge en fonction du débit aspiré pour l'enceinte 10 des figures 2a et 2b, d'un diamètre de 40mm, la flèche F2 indiquant le débit atteignable avec cette enceinte, qui est d'environ 440 litres par minute. La courbe C représente la variation de la perte de charge en fonction du débit aspiré pour l'enceinte 10 des figures 3a et 3b, d'un diamètre de 50mm, la flèche F3 correspondant au débit atteignable qui est d'environ 610 litres par minute. La courbe D représente la variation de la perte de charge en fonction du débit aspiré pour une enceinte 10 d'un diamètre de 60mm, le débit atteignable correspondant, indiqué par la flèche F4 étant d'environ 750 litres par minute.

De façon générale, l'efficacité de collecte est le produit de trois facteurs : l'efficacité de captation au niveau de l'aspiration, l'efficacité de séparation dans l'enceinte et l'efficacité de récupération dans le liquide contenu dans l'enceinte.

L'efficacité de captation et l'efficacité de séparation dépendent relativement peu de la taille des particules captées et des variations du débit d'aspiration. L'efficacité de récupération dans le liquide dépend de la qualité du rinçage des parois de l'enceinte 10, du volume de liquide contenu dans l'enceinte et de la vitesse de l'air dans l'enceinte. Il faut en particulier que cette vitesse reste comprise entre une limite inférieure et une limite supérieure, qui correspondent à des débits volumiques de 500 et 700 litres par minute environ, respectivement, pour une enceinte d'un diamètre de 50mm et de 700 et 1000 litres par minute, environ, respectivement, pour une enceinte d'un diamètre de 60mm.

Le volume de liquide contenu dans l'enceinte et la vitesse de l'air dans l'enceinte doivent être suffisants pour assurer le rinçage correct des parois de l'enceinte et doivent éviter ou au moins limiter l'entraînement de gouttes de liquide à l'extérieur de l'enceinte, car le liquide ainsi perdu contient des particules et des microorganismes qui ne peuvent être identifiés et dénombrés.

Pour éviter que les enceintes de plus grandes tailles, notamment celles d'un diamètre de 50mm et de 60mm, contiennent un volume de liquide trop important, il est possible de réduire la hauteur de la partie inférieure conique 16 de l'enceinte, comme indiqué schématiquement en figure 5, en la tronquant à son extrémité inférieure. Cela permet de réduire d'environ 10 à 15ml le volume de liquide contenu dans l'enceinte 10 pour un prélèvement, et donc de se rapprocher des volumes de liquide contenus dans les enceintes 10 de plus faibles dimensions.

Le volume de liquide utilisé dans l'enceinte 10 de la figure 5 devient ainsi comparable aux besoins, qui sont par exemple de 15ml, ce qui assure une efficacité de séparation et de récupération qui reste sensiblement constante lorsqu'on change d'enceinte.

Il est par ailleurs important de connaître avec précision la quantité d'air réellement aspiré en fin de prélèvement. Les résultats de dénombrement sont en effet toujours rapportés au volume d'air aspiré. Une mesure interne du débit peut être réalisée au moyen d'un capteur de pression 28 à faible coût, qui peut être branché sur la canne de prélèvement 30 (figure 6) qui relie l'élément 32 d'entrée et de sortie d'air coiffant l'enceinte 10 à un boîtier 34 contenant les moyens d'aspiration d'air, constitués ici par une hélice 36 entraînée en rotation par un moteur électrique 38. Dans l'exemple de la figure 6, l'élément 32 d'entrée et de sortie d'air est à entrée d'air omnidirectionnelle, l'aspiration de l'air se faisant autour de la partie supérieure cylindrique 14 de l'enceinte 10 à travers un filtre 40.

Des essais ont confirmé une bonne corrélation entre la perte de charge mesurée par le capteur 28 et le débit d'air aspiré. Il faut, de préférence, que le branchement du capteur 28 sur la canne de prélèvement 30 soit suffisamment éloigné en aval du coude de cette canne, la distance entre ce coude et le point de branchement du capteur 28 étant par exemple d'environ 10 fois le diamètre de la canne de prélèvement 30 et également que ce point de branchement ne soit pas trop près de l'entrée du boîtier 34, le capteur 28 devant en être écarté d'une distance correspond à trois ou quatre fois environ le diamètre de la canne de prélèvement 30.

La dépression au niveau du branchement est par exemple d'environ 100mbars, pour un débit de 500 litres par minute avec une enceinte 10 d'un diamètre de 40mm. Le capteur 28 utilisé est un capteur fournissant une mesure de pression différentielle, c'est-à-dire une mesure de la dépression par rapport à la pression atmosphérique ambiante. On peut également utiliser un capteur de pression absolue qui servira également à déterminer la pression atmosphérique ambiante avant le début du prélèvement.

Le boîtier 34 qui contient les moyens 36, 38 d'aspiration d'air, peut comporter un compartiment refroidi, par exemple au moyen d'un élément thermoélectrique à effet Peltier, contenant un flacon de recueil de l'échantillon liquide contenu dans la partie inférieure de l'enceinte 10. Cela permet de conserver l'échantillon liquide à une température basse prédéterminée, par exemple de 8°C, lorsque l'échantillon liquide n'est pas recueilli immédiatement après un prélèvement par un opérateur. Le compartiment refroidi est isolé thermiquement de l'environnement extérieur.

On peut également prévoir dans le boîtier 34 des moyens d'injection de liquide dans l'enceinte 10, soit avant de commencer un prélèvement, la quantité de liquide injecté correspondant alors au volume de l'échantillon liquide désiré en fin de prélèvement, soit en cours de prélèvement, pour compenser une évaporation du liquide pendant des prélèvements de durée relativement longue.

Ces moyens d'injection de liquide peuvent être amovibles et installés au niveau de la canne de prélèvement. Ils comprennent par exemple une pompe péristaltique reliant un flacon de liquide de prélèvement à une entrée de liquide prévue dans l'enceinte 10 ou dans la partie voisine de la canne de prélèvement 30. On peut aussi prévoir des moyens de mesure ou de détection du niveau de liquide dans l'enceinte, tels que des moyens à ondes sonar, une caméra, etc, ou encore utiliser des abaques enregistrées dans une mémoire du dispositif et donnant la quantité de liquide à injecter en fonction de la température et de l'hygrométrie du lieu de prélèvement.

Il est avantageux également, lorsqu'on réalise un prélèvement, d'ouvrir la sortie de l'enceinte quelques secondes avant la fin du prélèvement, quand l'air aspiré tourbillonne encore dans l'enceinte, car cela facilite l'écoulement du prélèvement hors de l'enceinte.

Le dispositif selon l'invention est commandé par des moyens informatiques du type PC (ordinateur personnel) ou PDA (assistant personnel) qui permettent d'enregistrer en mémoire les données relatives aux prélèvements réalisés (débits d'aspiration, durées des prélèvements, heures de début de prélèvement, volumes d'air réellement aspirés, température et hygrométrie, ...) et de faire un certain nombre de tests automatiques de vérification du dispositif et de son bon fonctionnement avant d'effectuer un prélèvement, tels notamment que :
- lecture d'une horloge temps réel et affichage de l'heure courante,
- vérification de la possibilité d'écriture et de lecture d'une mémoire de sauvegarde,
- lecture des capteurs de température et d'hygrométrie ambiantes,
- lecture du capteur de pression installé dans la canne de prélèvement,
- test de mise sous tension et de fonctionnement de la pompe d'injection de liquide,
- test de mise sous tension et de fonctionnement de l'électrovanne,
- affichage pendant quelques secondes du résultat des tests effectués,
- vérification et affichage du niveau de charge de la batterie électrique du dispositif.

D'autres informations sont également enregistrées avant chaque prélèvement ou série de prélèvements, telles que le nom de l'opérateur, le numéro de série du dispositif, le lieu de prélèvement, l'activité du lieu de prélèvement, etc, pour assurer la traçabilité des prélèvements effectués.

## Revendications

1. Dispositif de collecte et de séparation de particules et de microorganismes présents dans l'air ambiant, comprenant un ensemble d'enceintes amovibles de centrifugation (10) comportant chacune une entrée et une sortie d'air et formant un récipient de transport d'un échantillon liquide contenant les particules et microorganismes collectés, le dispositif comportant en outre des moyens d'aspiration d'air dans l'une desdites enceintes, **caractérisé en ce que** ladite enceinte (10) est sélectionnée dans l'ensemble d'enceintes du dispositif ayant des diamètres différents et permettant de réaliser, avec les mêmes moyens d'aspiration, des prélèvements avec des débits d'aspiration compris entre 100 et 2000 litres par minute environ.

2. Dispositif selon la revendication 1, **caractérisé en ce que** les diamètres des enceintes sont compris entre 20 et 100mm environ.

3. Dispositif selon la revendication 1 ou 2, **caractérisé en ce que**, les enceintes comprenant une partie supérieure cylindrique (14) et une partie inférieure conique (16), l'extrémité inférieure de la partie conique d'au moins la plus grande enceinte étant tronquée pour réduire le volume de liquide de prélèvement contenu dans cette enceinte.

4. Dispositif selon l'une des revendications précédentes, **caractérisé en ce que** l'enceinte (10) est raccordée par une canne de prélèvement (30) en forme de J à un boîtier (34) contenant les moyens (36, 38) d'aspiration d'air, ce boîtier pouvant être disposé horizontalement ou verticalement en position de service.

5. Dispositif selon l'une des revendications précédentes, **caractérisé en ce qu'**il comprend des moyens de détermination et/ou de contrôle de la quantité de liquide dans l'enceinte (10) et des moyens amovibles d'injection de liquide dans l'enceinte.

6. Dispositif selon l'une des revendications précédentes, **caractérisé en ce qu'**il comprend des moyens de mesure du débit d'air aspiré dans l'enceinte, ces moyens comprenant un capteur de pression (28) monté sur la canne de prélèvement (30) reliant l'enceinte (10) aux moyens d'aspiration d'air.

7. Dispositif selon la revendication 6, **caractérisé en ce que** le point de montage du capteur de pression (28) sur la canne de prélèvement (30) est éloigné du coude de la canne du côté relié à l'enceinte, et de l'entrée des moyens d'aspiration.

8. Dispositif selon l'une des revendications précédentes, **caractérisé en ce qu'**il comprend un flacon de recueil de prélèvement, placé à l'intérieur d'un compartiment refroidi, par exemple équipé d'un élément thermoélectrique à effet Peltier.

9. Dispositif selon la revendication 8, **caractérisé en ce que** l'enceinte (10) comprend à son extrémité inférieure des moyens (20) de vidange dans le flacon de recueil.

10. Dispositif selon l'une des revendications précédentes, **caractérisé en ce que** les enceintes (10) sont équipées d'éléments amovibles (12) d'entrée et de sortie d'air, les entrées d'air étant du type unidirectionnel ou omnidirectionnel.

11. Dispositif selon l'une des revendications précédentes, **caractérisé en ce qu'**il comprend des moyens informatiques de commande permettant d'enregistrer en mémoire des données relatives aux prélèvements effectués et de réaliser des tests automatiques de vérification du dispositif avant d'effectuer des prélèvements.

## Patentansprüche

1. Vorrichtung zum Sammeln und Trennen von in der Umgebungsluft vorhandenen Partikeln und Mikroorgansimen, enthaltend eine Einheit von abnehmbaren Zentrifugierkammern (10), die jeweils einen Lufteinlass und einen Luftauslass aufweisen und einen Transportbehälter zum Transportieren einer Flüssigprobe mit den gesammelten Partikeln und Mikroorganismen bilden, wobei die Vorrichtung ferner Luftansaugmittel zum Ansaugen von Luft in eine der Kammern aufweist, **dadurch gekennzeichnet, dass** die Kammer (10) aus der Einheit von Kammern der Vorrichtung ausgewählt ist, die unterschiedliche Durchmesser haben und gestatten, mit den gleichen Ansaugmitteln Entnahmen mit Ansaugdurchsatzmengen zwischen etwa 100 und 2000 Liter pro Minute gestatten.

2. Vorrichtung nach Anspruch 1, **dadurch gekennzeichnet, dass** die Durchmesser der Kammern zwischen etwa 20 und 100 mm betragen.

3. Vorrichtung nach Anspruch 1 oder 2, **dadurch gekennzeichnet, dass** die Kammern ein zylindrisches Oberteil (14) und ein konisches Unterteil (16) aufweisen, wobei das untere Ende des konischen Teils zumindest der größten Kammer kegelstumpfförmig ausgeführt ist, um das in dieser Kammer enthaltene Entnahmeflüssigkeitsvolumen zu vermindern.

4. Vorrichtung nach einem der vorangehenden Ansprüche, **dadurch gekennzeichnet, dass** die Kammer (10) über ein J-förmiges Entnahmerohr (30) mit einem Gehäuse (34) verbunden ist, das die Luftansaugmittel (36, 38) enthält, wobei dieses Gehäuse in Betriebsstellung horizontal oder vertikal angeordnet sein kann.

5. Vorrichtung nach einem der vorangehenden Ansprüche, **dadurch gekennzeichnet, dass** sie Mittel zum Ermitteln und/oder Kontrollieren der Flüssigkeitsmenge in der Kammer (10) und abnehmbare Mittel zum Einspritzen von Flüssigkeit in die Kammer enthält.

6. Vorrichtung nach einem der vorangehenden Ansprüche, **dadurch gekennzeichnet, dass** sie Messmittel zum Messen der in die Kammer angesaugten Luftdurchsatzmenge enthält, wobei diese Mittel einen Drucksensor (28) enthalten, der an dem Entnahmerohr (30) angebracht ist, welches die Kammer (10) mit den Luftansaugmitteln verbindet.

7. Vorrichtung nach Anspruch 6, **dadurch gekennzeichnet, dass** die Anbringstelle des Drucksensors (28) an dem Entnahmerohr (30) von der Biegung des Rohrs auf der mit der Kammer verbundenen Seite und von dem Einlass der Ansaugmittel entfernt liegt.

8. Vorrichtung nach einem der vorangehenden Ansprüche, **dadurch gekennzeichnet, dass** sie ein Entnahmesammelfläschchen enthält, das innerhalb eines gekühlten Fachs positioniert ist, das beispielsweise mit einem thermoelektrischen Element mit Peltier-Effekt ausgestattet ist.

9. Vorrichtung nach Anspruch 8, **dadurch gekennzeichnet, dass** die Kammer (10) an ihrem unteren Ende Entleerungsmittel (20) zur Entleerung in das Sammelfläschchen enthält.

10. Vorrichtung nach einem der vorangehenden Ansprüche, **dadurch gekennzeichnet, dass** die Kammern (10) mit abnehmbaren Elementen (12) zum Einlass und Auslass von Luft ausgestattet sind, wobei die Lufteinlässe unidirektional oder omnidirektional ausgeführt sind.

11. Vorrichtung nach einem der vorangehenden Ansprüche, **dadurch gekennzeichnet, dass** sie Informatiksteuermittel enthält, die gestatten, auf die erfolgten Entnahmen bezogene Daten abzuspeichern und automatische Tests zum Überprüfen der Vorrichtung vor dem Durchführen der Entnahmen durchzuführen.

## Claims

1. A device for collecting and separating particles and microorganisms present in ambient air, the device comprising a set of removable centrifuging enclosures (10), each of which has an air inlet and an air outlet, and forming a receptacle for transporting a liquid sample containing the collected particles and microorganisms, the device further comprising suction means for sucking air into one of said enclosures, the device being **characterized in that** said enclosure (10) is selected from the set of enclosures of the device having different diameters and suitable for enabling the same suction means to take samples at suction rates lying in the range about 100 liters per minute to about 2000 liters per minute.

2. A device according to claim 1, **characterized in that** the diameters of the enclosures lie in the range about 20 mm to about 100 mm.

3. A device according to claim 1 or claim 2, **characterized in that** the enclosures comprise a cylindrical top portion (14) and a conical bottom portion (16), the bottom end of the conical portion of at least the largest enclosure being truncated so as to reduce the volume of sample-taking liquid contained in said enclosure.

4. A device according to any preceding claim, **characterized in that** the enclosure (10) is connected by a J-shaped sample-taking pipe (30) to a chamber (34) containing the air suction means (36, 38), said chamber possibly being disposed horizontally or vertically in its operating position.

5. A device according to any preceding claim, **characterized in that** it includes means for determining and/or monitoring the quantity of liquid in the enclosure (10) and removable means for injecting liquid into the enclosure.

6. A device according to any preceding claim, **characterized in that** it includes means for measuring the flow rate of air sucked into the enclosure, said means comprising a pressure sensor (28) mounted on the sample-taking pipe (30) connecting the enclosure (10) to the air suction means.

7. A device according to claim 6, **characterized in that** the position where the pressure sensor (28) is mounted on the sample-taking pipe (30) is remote from the bend in the pipe at its end connected to the enclosure, and is remote from the inlet to the suction means.

8. A device according to any preceding claim, **characterized in that** it includes a sample collection flask placed inside a cooled compartment, e.g. fitted with a Peltier effect thermoelectric element.

9. A device according to claim 8, **characterized in that** the enclosure (10) includes means (20) at its bottom end for draining into a collection flask.

10. A device according to any preceding claim, **characterized in that** the enclosures (10) are fitted with removable air inlet and outlet elements (12), the air inlets being of the unidirectional or omnidirectional type.

11. A device according to any preceding claim, **characterized in that** it includes controlling computer means enabling data relating to the samples taken to be stored in memory and capable of performing automatic tests for verifying the device prior to taking samples.
